# EUROPEAN PATENT SPECIFICATION

(11) **EP 2 537 848 B1**
(45) Date of publication and mention of the grant of the patent: **08.04.2015**
(21) Application number: 11744673.2
(22) Date of filing: 16.02.2011
(51) Int. Cl.: C07D 471/04

(54) **METHOD FOR PRODUCING REDUCED PYRROLOQUINOLINE QUINONE**
VERFAHREN ZUR HERSTELLUNG VON REDUZIERTEM PYRROLOCHINOLINCHINON
PROCÉDÉ DE PRODUCTION DE PYRROLOQUINOLÉINE QUINONE RÉDUITE

(30) Priority: 16.02.2010 JP 2010031637
(43) Date of publication of application: 26.12.2012
(73) Proprietor: Mitsubishi Gas Chemical Company, Inc., Tokyo 100-8324 (JP)
(72) Inventor: IKEMOTO Kazuto, Niigata-shi Niigata 950-3112 (JP); NAKANO Masahiko, Niigata-shi Niigata 950-3112 (JP); EDAHIRO Junichi, Niigata-shi Niigata 950-3112 (JP)
(74) Representative: Müller-Boré & Partner Patentanwälte PartG mbB
(86) International application number: PCT/JP2011/053283
(87) International publication number: WO 2011/102387

(56) References cited:
- WO-A1-94/01142
- WO-A1-2005/035477
- JP-A- 2012 097 020
- GALLOP P M ET AL: "Acid-promoted tautomeric lactonization and oxidation-reduction of pyrroloquinoline quinone (PQQ)", BIOCHEMICAL AND BIOPHYSICAL RESEARCH COMMUNICATIONS, ACADEMIC PRESS INC. ORLANDO, FL, US, vol. 163, no. 2, 15 September 1989 (1989-09-15), pages 755-763, XP024769793, ISSN: 0006-291X, DOI: 10.1016/0006-291X(89)92287-0 [retrieved on 1989-09-15]
- ITOH, SHINOBU ET AL: "Oxidation of D-glucose by Coenzyme PQQ: 1,2-Enediolates as Substrates for PQQ Oxidation", JOURNAL OF THE CHEMICAL SOCIETY, CHEMICAL COMMUNICATIONS, CODEN: JCCCAT; ISSN: 0022-4936, no. 20, 1987, pages 1580-1581, XP002697817,
- GALLOP, P.M. ET AL.: 'Acid-promoted tautomeric lactonization and oxidation-reduction of pyrroloquinoline quinone (PQQ)' BIOCHEMICAL AND BIOPHYSICAL RESEARCH COMMUNICATIONS vol. 163, no. 2, 1989, pages 755 - 763
- KANO, K. ET AL.: 'Voltammetric determination of acid dissociation constants of pyrroloquinoline quinone and its reduced form under acidic conditions' BIOELECTROCHEMISTRY AND BIOENERGETICS vol. 24, no. 2, 1990, pages 193 - 201
- OUCHI, A. ET AL.: 'Kinetic study of the antioxidant activity of pyrroloquinolinequinol (PQQH2, a reduced form of pyrroloquinolinequinone) in micellar solution' JOURNAL OF AGRICULTURAL AND FOOD CHEMISTRY vol. 57, no. 2, 2009, pages 450 - 456
- ITOH, S. ET AL.: 'Reaction of reduced PQQ (PQQH2) and molecular oxygen' BULLETIN OF THE CHEMICAL SOCIETY OF JAPAN vol. 59, no. 6, 1986, pages 1911 - 1914
- DUINE, J.A. ET AL.: 'Characterization of the second prosthetic group in methanol dehydrogenase from Hyphomicrobium X' EUROPEAN JOURNAL OF BIOCHEMISTRY vol. 118, no. 2, 1981, pages 395 - 399

## Description

This application enjoys the benefit of Japanese Patent Application No. 2010-31637, filed on February 16, 2010.

This invention relates to a production method of reduced pyrroloquinoline quinone and reduced pyrroloquinoline quinone obtained thereby.

Pyrroloquinoline quinone (hereinafter sometimes referred to as "PQQ") has been proposed and has attracted much attention as a possible new vitamin (Non-patent document 1). Moreover, PQQ is present not only in bacteria but also in eukaryotic molds and yeasts and plays an important role as a coenzyme. Also, PQQ has been found to have many physiological activities such as cell growth-promoting activity, anti-cataract activity, hepatic disease-preventing and therapeutic activity, wound healing activity, antiallergic activity, reverse transcriptase-inhibiting activity, glyoxalase I-inhibiting activity-anticancer activity, and the like. The PQQs can be produced by processes such as organic chemical synthesis (Non-patent document 2) and fermentation (Patent document 1).

Reduced PQQ is reported to exhibit extremely higher antioxidative activity than that exhibited by the conventional PQQ (Non-patent document 3), and is a useful compound as foods with nutrient function claims, foods for specified health uses, nutrients, nutraceuticals, beverages, feedstuff, veterinarian medicine, cosmetics, pharmaceuticals, therapeutics, prophylactics, etc. Reduced PQQ is reported to be obtained by subjecting oxidized PQQ to reduction with common reducing agents such as sodium borohydride and sodium hyposulfite, reduction with hydrogen on a platinum catalyst, and reduction with glutathione (Non-patent document 3, 4, and 5). These common reducing agents are, however, likely to exhibit toxicity to living organisms. This requires a further step of removing such agents. Moreover, when a catalyst is used, special equipment is required because hydrogen, which readily escapes and is highly explosive, is used. The reduction with glutathione is safer and more easily used, but has a disadvantage in that glutathione is costly. There is therefore a need for a safe, convenient and low-cost method.

Ascorbic acid is well known as a substance that has reducing action on certain substances. Therefore, a combination of PQQ with ascorbic acid may be proposed as a composition that exhibits antioxidative activity. The reduction with ascorbic acid, however, has been thought to be difficult because the reduced form of PQQ exhibits greater antioxidative activity than that exhibited by ascorbic acid, as described in Non-patent document 3. Furthermore, reduced PQQ is readily oxidized to oxidized PQQ by molecular oxygen. This requires certain measures for storage of reduced PQQ.

### Patent document

Patent document 1 : Japanese Patent Application Laid-Open Publication N0.1-218597

### Non-patent document

Non-patent document 1: Nature, vol. 422, 24, April, 2003, 832
Non-patent document 2: JACS, vol. 103, 5599-5600 (1981)
Non-patent document 3: J. Agric. Food Chem. 2009, 57, 450-456
Non-patent document 4: Bull. Chem. Soc. Jpn, 59, 1911-1914 (1986)
Non-patent document 5: Eur. J. Biochem. 118, 395-399 (1981)

Further, WO 94/01142 A1 discloses a physiologically acceptable therapeutic mixture comprising PQQ and at least one reducing agent wherein a net reducing capacity greater than the oxidizing capacity of the PQQ in said mixture, said reducing agent or agents being present in substantial molar excess of said PQQ, and wherein said reducing agent may be ascorbate. In P. M. Gallop et al., Biochem. Biophys. Res. Commun. 163, 755-763, the acid-promoted tautomeric lactonization and oxidation-reduction of pyrroloquinoline quinone is described. A study on the oxidation of D-glucose and related substrates by coenzyme PQQ is described in S. Itoh et al., J. Chem. Soc., Chem. Commun. 20, 1580-1581. In K. Kano et al., Bioelectrochem. Bioenerg. 24, 193-201, voltammetric determination of acid dissociation constants of pyrroloquinoline quinone and its reduced form under acidic conditions are described. Further, US 2005/0092969 A1 discloses a method of stabilizing a compound having a quinone skeleton in a composition comprising a compound having a quinone skeleton and an antioxidant which comprises coating at least one of said compound having a quinone skeleton and an antioxidant, either with an oil-insoluble coating medium to make both constituents coexist in an oily substance, or coating at least one of said compound having a quinone skeleton and an antioxidant with a water-insoluble coating medium to make both constituents coexist in an aqueous substance.

The present inventors have found that reduced pyrroloquinoline quinone is obtained in high yield from a solution having a pH of 3.5 or less obtained by mixing a solution of pyrroloquinoline quinone having a pH of 4 or less with a solution of ascorbic acid. The present invention is based on this finding.

An object of the present invention is to provide a safe, convenient and low-cost production method of reduced pyrroloquinoline quinone and reduced pyrroloquinoline quinone obtained thereby.

According to the present invention, the following inventions are provided:
(1) a method for producing reduced pyrroloquinoline quinone, comprising a step of mixing an aqueous solution of pyrroloquinoline quinone or a salt thereof having a pH adjusted to 4 or less with an ascorbic acid analog to obtain a solution having a pH of 3.5 or less comprising reduced pyrroloquinoline quinone, wherein the ascorbic acid analog is selected from the group consisting of ascorbic acid, rhamno-ascorbic acid, arabo-ascorbic acid, gluco-ascorbic acid, fuco-ascorbic acid, glucohepto-ascorbic acid, xylo-ascorbic acid, galacto-ascorbic acid, gulo-ascorbic acid, allo-ascorbic acid, erythro-ascorbic acid, 6-desoxyascorbic acid, and an ester and a salt thereof;
(2) the production method of (1), wherein the solution of the pyrroloquinoline quinone or salt thereof has a pH of 2 to 3.5;
(3) the production method of (1), wherein the molar ratio of the ascorbic acid analog with respect to the pyrroloquinoline quinone or salt thereof is 1:0.5 to 1000;
(4) the production method of (1), further comprising a step of separating the reduced pyrroloquinoline quinone from the solution.

Advantageously, according to the present invention, reduced pyrroloquinoline quinone can be produced safely, conveniently and at low cost, and no expensive equipment is required in producing reduced pyrroloquinoline quinone. Also advantageously, according to the present invention, reduced pyrroloquinoline quinone can be stored stably.

According to the present invention, reduced pyrroloquinoline quinone can be produced by mixing a solution of pyrroloquinoline quinone or a salt thereof having a pH of 4 or less with an ascorbic acid analog to obtain a solution having a pH of 3.5 or less.

The term "reduced pyrroloquinoline quinone" as used herein refers to the compound represented by formula (1).

### [Compound 1]

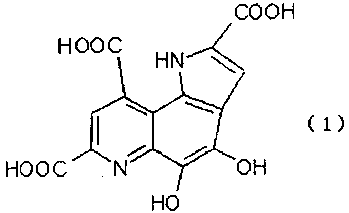

Pyrroloquinoline quinone (in the free form) used in the present invention refers to the compound represented by formula (2).

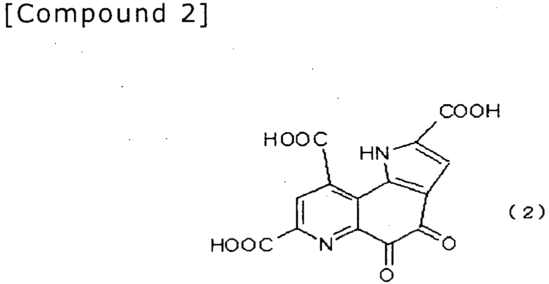

According to the present invention, reduced PQQ can be produced by reducing pyrroloquinoline quinone with an ascorbic acid analog.

Pyrroloquinoline quinone used in the present invention can be pyrroloquinoline quinone (in the free form) or a salt of pyrroloquinoline quinone.

The "salt of pyrroloquinoline quinone" used in the present invention includes an alkali metal salt, an alkaline-earth metal salt, and an ammonium salt of pyrroloquinoline quinone, with alkali metal salt being preferred.

Pyrroloquinoline quinone used in the present invention can be in the free form or in the form of any alkali metal salt. Readily available pyrroloquinoline quinone, in the free form and in the form of disodium and dipotassium salts, among others, is easily used.

The alkali metal salt of pyrroloquinoline quinone used in the present invention includes salts of sodium, potassium, lithium, cesium, rubidium, and the like. Preferably, sodium and potassium salt are more preferred because they are readily available. Pyrroloquinoline quinone may be substituted with one to three atoms of alkali metals to form an alkali metal salt thereof, which may be any of a monoalkali metal salt, a dialkali metal salt and a trialkali metal salt, preferably a dialkali metal salt. The alkali metal salt of pyrroloquinoline quinone is especially preferably disodium and dipotassium salts.

Pyrroloquinoline quinone or a salt thereof used in the present invention may be commercially available or produced by known methods.

Pyrroloquinoline quinone or a salt thereof used in the present invention is used as an aqueous solution of pyrroloquinoline quinone or a salt thereof.

The solution of pyrroloquinoline quinone or a salt thereof can be prepared in a concentration of, for example, 0.001 to 30 g/L, preferably 0.01 to 15 g/L, more preferably 0.1 to 5 g/L.

The pH of the solution of pyrroloquinoline quinone or a salt thereof can be adjusted to 4 or less in order to efficiently obtain reduced PQQ. Also, the pH can be adjusted to 1 or more in order to increase the solubility of pyrroloquinoline quinone. The pH of the solution of pyrroloquinoline quinone or a salt thereof is preferably 1 to 4, more preferably 1 to 3.5, even more preferably 2 to 3.5, and even more preferably 2 to 2.5. To adjust the pH of the solution, an acidic substance (for example, hydrochloric acid) or an alkaline substance (for example, sodium hydroxide) can be used.

The ascorbic acid analog includes ascorbic acid, and those analogous to ascorbic acid such as rhamno-ascorbic acid, arabo-ascorbic acid, gluco-ascorbic acid, fuco-ascorbic acid, glucohepto-ascorbic acid, xylo-ascorbic acid, galacto-ascorbic acid, gulo-ascorbic acid, allo-ascorbic acid, erythro-ascorbic acid, 6-desoxyascorbic acid as well as esters and salts thereof (for example, palmitate, stearate, sodium salt, and calcium salt). Moreover, these ascorbic acid analogs may be in L- (for example, L-ascorbic acid and sodium L-ascorbate), D- (for example, D-arabo-ascorbic acid, sodium D-arabo-ascorbate), or racemic form.

More specifically, the analogs may include, for example, L-ascorbic acid, L-ascorbic acid palmitate, L-ascorbic acid stearate, and D-arabo-ascorbic acid.

Any of the ascorbic acid analogs mentioned above can be suitably used in the production of reduced pyrroloquinoline quinone, and among the ascorbic acid analogs mentioned above, particularly suitably used are water-soluble analogs in consideration of the ease of separation from the reduced pyrroloquinoline quinone produced. Most preferred are the analogs in the free form such as L-ascorbic acid and D-arabo-ascorbic acid from the viewpoint of availability and cost.

The ascorbic acid analog used in the present invention may be commercially available or produced by known methods.

The ascorbic acid analog used in the present invention can be used neat or in a solution. When used in solution, the ascorbic acid analog can be dissolved in a solvent such as water, alcohol, and dimethyl sulfoxide to form a solution. Preferably, the ascorbic acid analog is used as an aqueous solution.

The solution of the ascorbic acid analog can be prepared in a concentration of, for example, 0.1 to 500 g/L, preferably 0.5 to 100 g/L.

In the production method according to the present invention, the step of "mixing an aqueous solution of pyrroloquinoline quinone or a salt thereof with an ascorbic acid analog to obtain a solution having a pH of 3.5 or less comprising reduced pyrroloquinoline quinone" can be accomplished by reacting pyrroloquinoline quinone or a salt thereof with an ascorbic acid analog in a solvent, such as, for example, by mixing a solution of pyrroloquinoline quinone or a salt thereof with a solution of an ascorbic acid analog, or adding an ascorbic acid analog to a solution of pyrroloquinoline quinone or a salt thereof to reduce the pyrroloquinoline quinone, resulting in reduced pyrroloquinoline quinone.

In the step mentioned above, also included is an embodiment where a solution comprising pyrroloquinoline quinone or a salt thereof and an ascorbic acid analog is orally administered to a mammal, and the reaction is allowed to occur in vivo (for example, oral cavity, stomach, and intestine).

"Mixing" as used herein can be accomplished by adding one object to be mixed to the other object to be mixed, or adding the objects to be mixed to a separate vessel.

For "adding" as used herein, an additive may be added at once or gradually to an object to which the additive is to be added.

In this reaction, the molar ratio of pyrroloquinoline quinone or a salt thereof to an ascorbic acid analog may vary depending on the content of the desired reduced form of PQQ, assuming an equimolar reaction of pyrroloquinoline quinone or a salt thereof with an ascorbic acid analog. In practical terms, the ascorbic acid analog is preferably used in an amount of 0.5 to 1000-fold moles with respect to PQQ, and in the case of isolation of reduced PQQ, preferably 0.9 to 10-fold moles. When the ascorbic acid analog is used in an amount lower than this range, the content of the reduced form of PQQ is low, so that no desired effect can be produced. Using the ascorbic acid analog in an amount beyond the range may not cause trouble but leads to higher cost.

In the production method according to the present invention, the molar ratio of an ascorbic acid analog with respect to pyrroloquinoline quinone or a salt thereof may be, in a solvent, 1:0.5 to 1000, preferably 1:1.5 to 1000, more preferably 1:1.5 to 100, and even more preferably 1:1.5 to 10.

In the production method according to the present invention, the reaction can be carried out at any temperature, for example, a temperature of -10°C to 180°C, and preferably of 0°C to 100°C. The reaction proceeds even at room temperature. Preferably, if the rate of reaction is to be increased, then the temperature is raised, and in order to increase the throughput, preferred is a temperature of 50°C or more, due to higher solubility at higher temperature.

In the production method according to the present invention, the reaction can be carried out for any period of time, for example, for 0.2 to 48 hours, and preferably for 0.5 to 24 hours.

The reaction may be carried out in any solvent that allows the reaction to proceed, and preferably in an aqueous solution that is not highly problematic even when it remains in the product. The reaction is carried out particularly in an aqueous solution having a pH of 5 or less, and more preferably of 4 or less, since the reaction may not proceed easily under alkaline conditions. It is not particularly problematic that acid or alkaline is used to adjust the pH of the solution. Acid or alkaline may be used, if necessary.

In the production method according to the present invention, the reaction is not particularly limited, but is preferably carried out in a low oxygen atmosphere. To prevent oxygen in the air from converting reduced PQQ into oxidized PQQ, the operation mentioned above can be carried out in an inert gas atmosphere such as nitrogen and argon according to conventional methods. Reduction of the oxidation reaction can be achieved by replacement with inert gas, reduced pressure, boiling, or any combination thereof. It is advisable to at least use replacement with inert gas, i.e. an inert gas atmosphere. The inert gas mentioned above can include, for example, nitrogen gas, helium gas, argon gas, hydrogen gas, carbonic acid gas and the like, and preferred is nitrogen gas.

In the production method according to the present invention, the pH of a solution (reaction solution) obtained by reacting a solution of pyrroloquinoline quinone or a salt thereof with an ascorbic acid analog can be adjusted to 3.5 or less by adjustment of the pH of the solution of pyrroloquinoline quinone or a salt thereof and the amount of the ascorbic acid analog, or through a pH adjustment step using an acidic substance (for example, hydrochloric acid) or an alkaline substance (for example, sodium hydroxide).

The pH of the resultant solution can be adjusted to 3.5 or less, preferably 3 or less, more preferably 2.8 or less, and even more preferably 2.6 or less.

More specifically, the production method according to the present invention may be carried out as follows:

Pyrroloquinoline quinone is prepared in an aqueous solution having a concentration of 0.01 g/L to 15 g/L. This concentration range is described as a range in which pyrroloquinoline quinone is dissolved, and beyond this range, the reaction tends to proceed in suspension. The reaction is completed by a simple procedure of adding the ascorbic acid in powder or solution thereto. The reaction temperature is usually from 0°C to 100°C. The reaction time varies depending on the reaction temperature, and a reaction time of about 0.2 to about 48 hours is easily used.

At this time, acid or base may be added if the pH is to be adjusted.

In the solution obtained through the reaction, the reduced form of PQQ is eventually precipitated. The resultant precipitate can be reduced pyrroloquinoline quinone.

The term "precipitate" as used herein refers to a solid phase (solid) formed from a liquid phase (solution).

The precipitate can be separated from the solution. The precipitate (the reduced form of PQQ precipitated) can be obtained by filtration, centrifugation, or decantation. In addition, the obtained precipitate may also be washed with water or alcohol. It may also be then dried under reduced pressure to obtain a solid product. Alternatively, the precipitate can be provided as is, without such separation operations.

In addition, in the case where a purer substance is required, recrystallization enables the purity of the substance to be improved. Recrystallization may be carried out by operations of dissolving the substance in a good solvent such as dimethyl sulfoxide, and reducing the solubility of the solution, for example, by lowering the temperature of the solution, adding a poor solvent to the solution, or concentrating the solution. In addition, the substance can also be purified by column chromatography.

In the production method of reduced pyrroloquinoline quinone according to the present invention, the reaction proceeds well at near room temperature. Therefore, a composition containing 0.5 to 1000-fold moles of an ascorbic acid analog with respect to the number of moles of the pyrroloquinoline quinone or salt thereof can be provided to produce reduced pyrroloquinoline quinone in a vessel including a cup immediately before intake, or in the oral cavity, stomach, and intestine in vivo.

A composition containing 0.5 to 1000-fold moles of an ascorbic acid analog with respect to the number of moles of the pyrroloquinoline quinone or salt thereof can be made to use for the purposes mentioned above, since the reaction proceeds even at room temperature, which is consistent with the object of the present invention. If this composition, whether in a solid form or a solution form, is orally administered, then the composition turns to a solution state and reacts.

According to a preferred embodiment of the production method of the present invention, there is provided a production method of reduced pyrroloquinoline quinone, comprising a step of mixing a solution of pyrroloquinoline quinone or a salt thereof having a pH of 2 to 3.5 with an ascorbic acid analog or a solution thereof to obtain a solution having a pH of 3 or less comprising reduced pyrroloquinoline quinone.

According to a more preferred embodiment of the production method of the present invention, there is provided a production method of reduced pyrroloquinoline quinone, comprising a step of mixing a solution of pyrroloquinoline quinone or a salt thereof having a pH of 2 to 2.5 with an ascorbic acid analog or a solution thereof to obtain a solution having a pH of 2.6 or less comprising reduced pyrroloquinoline quinone.

According to the present invention, there is provided reduced pyrroloquinoline quinone produced by the production method according to the present invention.

Reduced pyrroloquinoline quinone is susceptible to oxidation, and strict control to prevent its oxidation is therefore necessary for storage after separation and purification. It may be stored in a reducing atmosphere to prevent oxidation. However, conventional reducing agents are often toxic and are unable to be used. In the present invention, oxidation can be prevented by forming a composition containing ascorbic acid in molar ratios of 0.5 to 1000 with respect to reduced pyrroloquinoline quinone, which enables stable storage without performing purification and provision as it is. This is one of advantages of the production method of the present invention.

According to the present invention, to store reduced pyrroloquinoline quinone stably, reduced pyrroloquinoline quinone can be provided as a solution containing an ascorbic acid analog.

Also, after the separation operation, an ascorbic acid analog can be added to form a composition. This composition can be provided in a state of a solid, or suspension or solution, and the composition in suspension in water can be easily provided based on the reaction conditions.

The reduced pyrroloquinoline quinone obtained by the present invention may take any dosage form, which can be selected appropriately depending on use applications. A composition for oral ingestion of the present invention can be used for humans or animals as foods, functional foods, pharmaceuticals or quasi drugs. Functional foods as used here mean foods taken for the purpose of maintenance of health or nutrition as an alternative to meals, such as health foods, nutrients, foods with nutrient function claims, and nutrient health food. Specific dosage forms include, but are not limited to, capsules, tablets, chewables, tablets, and drinks.

In the commercialization as functional foods, additives used for foods, for example, sweeteners, colorants, preservatives, thickening agents, antioxidants, color fixatives, bleaching agents, antibacterial and antifungal agents, gum bases, bittering agents, enzymes, glazing agents, acidifiers, seasoning, emulsifiers, enrichments, manufacturing agents, flavoring agents, spice extracts and the like can be used. It is generally possible to add to usual foods, for example, miso, soy sauce, instant miso soup, ramen, yakisoba, curry, corn soup, mabo-dofu, mabo-nasu, pasta sauce, pudding, cake, bread and the like.

### EXAMPLES

The present invention will now be described more specifically with reference to the following examples and comparative examples, but is not intended to be limited thereto. In addition, all percentages in the context of the present invention are by weight, unless otherwise stated.

In Examples and Comparative Examples, the ¹³C-NMR measurements were performed on a JEOL 500 MHz NMR, JNM-ECA500 Spectrometer at room temperature.

In Examples and Comparative Examples, the UV measurements were performed on a HITACHI U-2000 Spectrophotometer.

### Confirmatory Experiment for Reduction Reaction

### Example 1

A reagent (trade name: BioPQQ) from MITSUBISHI GAS CHEMICAL COMPANY, INC. was used as a raw material pyrroloquinoline quinone disodium. L-ascorbic acid was available from Wako Pure Chemical Industries, Ltd.

In 500 g of water, 1.53 g of pyrroloquinoline quinone disodium was dissolved to a concentration of 0.01 mol/L. In 100 g of water, 3.51 g of L-ascorbic acid was dissolved to a concentration of 0.2 mol/L.

20 g of a pyrroloquinoline quinone disodium solution having a concentration of 0.01 mol/L (pH 3.5) was mixed with 20 g of an L-ascorbic acid solution having a concentration of 0.2 mol/L (pH 2.5). The molar ratio of L-ascorbic acid is 20 with respect to pyrroloquinoline quinone. These two solutions were mixed at room temperature, and reacted at 70°C for 2 hours (pH 2.5). After the reaction, a solid was precipitated. To this solution, hydrochloric acid was added to adjust the value of pH to 1 or less. This was centrifuged at 1000 rpm for 10 minutes, and the supernatant was removed to obtain a solid. The resultant solid was washed with a degassed aqueous hydrochloric acid solution, and dried under nitrogen gas stream. Heavy dimethyl sulfoxide was added thereto, and then this solution was transferred to an NMR tube under nitrogen gas stream, and the ¹³C-NMR measurements were performed at room temperature.

The results obtained were as follows: 105.7, 111.0, 119.4, 122.9, 123.6, 128.1, 131.3, 134.2, 137.8, 140.9, 142.6, 162.2, 165.5, 170.1 ppm (DMSO-d6: reference 39.5 ppm).

These values are in agreement with those for the reduced form described in non-patent document 5 and formation of the reduced form was confirmed. Moreover, peaks at 173.3 and 178.0 ppm attributable to the quinone structure were absent among these measured data.

### Comparative Example 1

To a saturated aqueous solution of pyrroloquinoline quinone disodium, hydrochloric acid was added to adjust the value of pH to 1 or less. There was a red solid precipitated, which was passed through a filter to obtain pyrroloquinoline quinone in the free form. Heavy dimethyl sulfoxide was added thereto, and then this solution was transferred to an NMR tube, and the ¹³C-NMR measurements were performed at room temperature.

The results obtained were as follows: 113.5, 124.5, 126.4, 127.6, 129.2, 134.3, 136.3, 146.8, 148.7, 160.9, 164.9, 168.7, 173.3, 178.0 ppm (DMSO-d6: reference 39.5 ppm). Unlike the peaks in Example 1, no peaks were observed that should appear for the reduced PQQ.

### Example 2

20 g of a pyrroloquinoline quinone disodium solution having a concentration of 0.01 mol/L (pH 3.5) was mixed with 10 g of an L-ascorbic acid solution having a concentration of 0.2 mol/L (pH 3). The molar ratio of L-ascorbic acid is 10 with respect to pyrroloquinoline quinone. These two solutions were mixed and stirred at room temperature. The color of the mixture immediately changed, and a solid was precipitated. After allowing the solution to stand at room temperature overnight, it had a pH of 3 as measured with a pH test strip. The solid was separated through centrifugation (1000 rpm, 10 minutes), and dried at reduced pressure to obtain 0.073 g of a solid of the reduced form containing water free from the oxidized form. Analysis of UV spectra showed that the solid was in the reduced state.

### Example 3

20 g of a pyrroloquinoline quinone disodium solution having a concentration of 0.01 mol/L (pH 3.5) was mixed with 5 g of an L-ascorbic acid solution having a concentration of 0.2 mol/L (pH 3). The molar ratio of L-ascorbic acid is 5 with respect to pyrroloquinoline quinone. These two solutions were mixed and stirred at room temperature. The color of the mixture immediately changed, and a solid was precipitated. After allowing the solution to stand at room temperature overnight, it had a pH of 3 as measured with a pH test strip. The solid was separated through centrifugation (1000 rpm, 10 minutes), and dried at reduced pressure to obtain 0.044 g of a solid of the reduced form. The isolated crude yield was 66%.

### Example 4

20 g of a pyrroloquinoline quinone disodium solution having a concentration of 0.001 mol/L (pH 3.5) was mixed with 10 g of an L-ascorbic acid solution having a concentration of 0.2 mol/L (pH 2). The molar ratio of L-ascorbic acid is 100 with respect to pyrroloquinoline quinone. These two solutions were mixed and stirred at room temperature. The color of the mixture immediately changed, and a solid was precipitated. After allowing the solution to stand at room temperature overnight, it had a pH of 2 as measured with a pH test strip. The solid was separated through centrifugation (1000 rpm, 10 minutes), and dried at reduced pressure to obtain 0.006 g of a solid of the reduced form.

### Example 5

0.5 g of a pyrroloquinoline quinone disodium solution having a concentration of 0.01 mol/L (pH 3.5) was mixed with 0.5 g of a sodium L-ascorbate solution having a concentration of 0.5 mol/L. The pH of the solution at this time was in a range of 6 to 7. The molar ratio of L-ascorbic acid is 50 with respect to pyrroloquinoline quinone. These two solutions were mixed at room temperature, and this mixed solution was added to 10 ml of simulated gastric fluid (pH 1.2). After allowing the solution to stand at room temperature overnight, a solid of the reduced form was precipitated. By bringing the solution into acidic conditions, the reaction tends to proceed easily. Also, it is considered that the reduction takes place even in vivo.

### Example 6

1 g of a pyrroloquinoline quinone disodium solution having a concentration of 0.01 mol/L (pH 3.5) was mixed with 1 g of an L-ascorbic acid solution having a concentration of 0.011 mol/L (pH 2.5). The molar ratio is 1:1.1 in this case. After the reaction at 30°C for eight days (pH 2.5), 82% of PQQ reacted and a substance containing the reduced form was obtained.

### Comparative Example 2

0.5 g of a pyrroloquinoline quinone disodium solution having a concentration of 0.01 mol/L was mixed with 0.5 g of a sodium L-ascorbate solution having a concentration of 0.5 mol/L at room temperature. The pH of the solution was in the range of 6 to 7 in this case. The molar ratio of L-ascorbic acid is 50 with respect to pyrroloquinoline quinone. When the mixed solution was allowed to stand at room temperature for three days, the solution did not change (pH 7), and the reduced form was not obtained.

### Storage Test

### Example 7

In 500 g of water, 1.53 g of pyrroloquinoline quinone disodium was dissolved to a concentration of 0.01 mol/L. In 100 g of water, 3.51 g of L-ascorbic acid was dissolved to a concentration of 0.2 mol/L.

20 g of the pyrroloquinoline quinone disodium solution mentioned above having a concentration of 0.01 mol/L (pH 3.5) was mixed with 20 g of an L-ascorbic acid solution having a concentration of 0.2 mol/L at room temperature. The molar ratio of L-ascorbic acid is 20 with respect to pyrroloquinoline quinone. The pH of the solution was 2 as measured with a pH test strip. The mixed solution was allowed to react at room temperature overnight (pH 2). After the reaction, a solid of the reduced form was precipitated. Under this condition, it is believed that at least 15-fold moles of L-ascorbic acid is present, even allowing for the amounts consumed in the reduction reaction and in air oxidization. Subsequently, after the solution was stored at room temperature for one week, it was maintained in the reduced state.

### Comparative Example 3

The solid of the reduced form obtained in Example 6 was separated through centrifugation. The supernatant was removed, and the solid was washed with water. This process removed L-ascorbic acid. An aqueous sodium hydroxide solution was added thereto to adjust the value of pH to 7. All the reduced form was converted to the oxidized form in 30 minutes.

### Example 8

1 g of a pyrroloquinoline quinone disodium solution having a concentration of 0.01 mol/L (pH 3.5) was mixed with 1 g of an L-ascorbic acid solution having a concentration of 0.014 mol/L. When 1 g of this mixed solution was transferred to a 15-ml container and brought into contact with air, the solution included 14% of oxidized PQQ (pH 3). After this oxidized PQQ-containing reduced PQQ was left at 30°C for one week, 18% of oxidized PQQ was included.

### CONFIRMATORY EXPERIMENT FOR PH CONDITIONS

### Example 9

Pyrroloquinoline quinone disodium was dissolved in water and the concentration was adjusted to 2 g/L. The pH of the aqueous solution was adjusted to 1 or less by using hydrochloric acid and NaOH. Also, L-ascorbic acid was dissolved in water and the concentration was adjusted to 4 g/L.

A 0.5 mL aliquot of each of the two aqueous solutions mentioned above was mixed in a 1.5-mL container, and the resultant mixture was reacted at room temperature for 24 hours. The pH after the reaction was 1. Subsequently, the resultant reaction solution was subjected to centrifugation (1000 rpm, 10 minutes), and the supernatant was removed to obtain a solid. The resultant solid was washed using hydrochloric acid having a concentration of about 9%, and dissolved in dimethyl sulfoxide. UV measurements were performed and the yield of the reduced form was calculated to be 68%.

### Example 10

Pyrroloquinoline quinone disodium was dissolved in water and the concentration was adjusted to 2 g/L. The pH of the aqueous solution was adjusted to 2.2 by using hydrochloric acid and NaOH. Also, L-ascorbic acid was dissolved in water and the concentration was adjusted to 4 g/L.

A 0.5 mL aliquot of each of the two aqueous solutions mentioned above was mixed in a 1.5-mL container, and the resultant mixture was reacted at room temperature for 24 hours. The pH after the reaction was 2.6. Subsequently, the resultant reaction solution was subjected to centrifugation (1000 rpm, 10 minutes), and the supernatant was removed to obtain a solid. The resultant solid was washed with hydrochloric acid having a concentration of about 9%, and dissolved in dimethyl sulfoxide. UV measurements were performed and the yield of the reduced form was calculated to be 100%.

### Example 11

Pyrroloquinoline quinone disodium was dissolved in water and the concentration was adjusted to 2 g/L. The pH of the aqueous solution was adjusted to 2.6 using hydrochloric acid and NaOH. Also, L-ascorbic acid was dissolved in water and the concentration was adjusted to 4 g/L.

A 0.5 mL aliquot of each of the two aqueous solutions mentioned above was mixed in a 1.5-mL container, and the resultant mixture was reacted at room temperature for 24 hours. The pH after the reaction was 3. Subsequently, the resultant reaction solution was subjected to centrifugation (1000 rpm, 10 minutes), and the supernatant was removed to obtain a solid. The resultant solid was washed using hydrochloric acid having a concentration of about 9%, and dissolved in dimethyl sulfoxide. UV measurements were performed and the yield of the reduced form was calculated to be 66%.

### Example 12

Pyrroloquinoline quinone disodium was dissolved in water and the concentration was adjusted to 2 g/L. The pH of the aqueous solution was adjusted to 3.5 using hydrochloric acid and NaOH. Also, L-ascorbic acid was dissolved in water and the concentration was adjusted to 4 g/L.

A 0.5 mL aliquot of each of the two aqueous solutions mentioned above was mixed in a 1.5-mL container, and the resultant mixture was reacted at room temperature for 24 hours. The pH after the reaction was 3.5. Additionally, 100 µL of concentrated hydrochloric acid was added to bring the pH of the solution to 1. Subsequently, the resultant mixed solution was subjected to centrifugation (1000 rpm, 10 minutes), and the supernatant was removed to obtain a solid. The resultant solid was washed using hydrochloric acid having a concentration about 9%, and dissolved in dimethyl sulfoxide. UV measurements were performed and the yield of the reduced form was calculated to be 54%.

### Comparative Example 4

Pyrroloquinoline quinone disodium was dissolved in water and the concentration was adjusted to 2 g/L. The pH of the aqueous solution was adjusted to 4.3 using hydrochloric acid and NaOH. Also, L-ascorbic acid was dissolved in water and the concentration was adjusted to 4 g/L.

A 0.5 mL aliquot of each of the two aqueous solutions mentioned above was mixed in a 1.5-mL container, and the resultant mixture was reacted at room temperature for 24 hours. The pH after the reaction was 3.5. Subsequently, the resultant reaction solution was subjected to centrifugation (1000 rpm, 10 minutes), and the supernatant was removed to obtain a solid. The resultant solid was washed using hydrochloric acid having a concentration of about 9%, and dissolved in dimethyl sulfoxide. UV measurements were performed and the yield of the reduced form was calculated to be 1%.

### Comparative Example 5

Pyrroloquinoline quinone disodium was dissolved in water and the concentration was adjusted to 2 g/L. The pH of the aqueous solution was adjusted to 7.6 using hydrochloric acid and NaOH. Also, L-ascorbic acid was dissolved in water and the concentration was adjusted to 4 g/L.

A 0.5 mL aliquot of each of the two aqueous solutions mentioned above was mixed in a 1.5-mL container, and the resultant mixture was reacted at room temperature for 24 hours. The pH after the reaction was 3.5. Subsequently, the resultant reaction solution was subjected to centrifugation (1000 rpm, 10 minutes), and the supernatant was removed to obtain a solid. The resultant solid was washed using hydrochloric acid having a concentration of about 9%, and dissolved in dimethyl sulfoxide. UV measurements were performed and the yield of the reduced form was calculated to be 9%.

## Claims

1. A method for producing reduced pyrroloquinoline quinone, comprising a step of mixing an aqueous solution of pyrroloquinoline quinone or a salt thereof having a pH adjusted to 4 or less with an ascorbic acid analog to obtain a solution having a pH of 3.5 or less comprising reduced pyrroloquinoline quinone, wherein the ascorbic acid analog is selected from the group consisting of ascorbic acid, rhamno-ascorbic acid, arabo-ascorbic acid, gluco-ascorbic acid, fuco-ascorbic acid, glucohepto-ascorbic acid, xylo-ascorbic acid, galacto-ascorbic acid, gulo-ascorbic acid, allo-ascorbic acid, erythro-ascorbic acid, 6-desoxyascorbic acid, and an ester and a salt thereof.

2. The method according to claim 1, wherein the solution of pyrroloquinoline quinone or a salt thereof has a pH of 2 to 3.5.

3. The method according to claim 1, wherein the molar ratio of the ascorbic acid analog with respect to the pyrroloquinoline quinone or salt thereof is 1:0.5 to 1000.

4. The method according to claim 1, further comprising a step of separating the reduced pyrroloquinoline quinone from the solution.

## Patentansprüche

1. Verfahren zur Herstellung von reduziertem Pyrrolochinolinchinon, umfassend einen Schritt des Mischens einer wässrigen Lösung von Pyrrolochinolinchinon oder einem Salz davon mit einem auf 4 oder weniger eingestellten pH-Wert mit einem Ascorbinsäure-Analogon, um eine Lösung mit einem pH-Wert von 3,5 oder weniger zu erhalten, umfassend reduziertes Pyrrolochinolinchinon,
wobei das Ascorbinsäure-Analogon aus der Gruppe, bestehend aus Ascorbinsäure, Rhamnoascorbinsäure, Araboascorbinsäure, Glucoascorbinsäure, Fucoascorbinsäure, Glucoheptoascorbinsäure, Xyloascorbinsäure, Galactoascorbinsäure, Guloascorbinsäure, Alloascorbinsäure, Erythroascorbinsäure, 6-Desoxyascorbinsäure und einem Ester und einem Salz davon, ausgewählt ist.

2. Verfahren nach Anspruch 1, wobei die Lösung von Pyrrolochinolinchinon oder einem Salz davon einen pH-Wert von 2 bis 3,5 aufweist.

3. Verfahren nach Anspruch 1, wobei das Molverhältnis des Ascorbinsäure-Analogons bezüglich des Pyrrolochinolinchinons oder eines Salzes davon 1:0,5 bis 1000 beträgt.

4. Verfahren nach Anspruch 1, weiter umfassend einen Schritt des Trennens des reduzierten Pyrrolochinolinchinons von der Lösung.

## Revendications

1. Méthode de production d'une pyrroloquinoléine quinone réduite, comprenant une étape de mélange d'une solution aqueuse de pyrroloquinoléine quinone ou d'un sel de celle-ci ayant un pH ajusté à 4 ou moins avec un analogue d'acide ascorbique pour obtenir une solution ayant un pH de 3,5 ou moins comprenant une pyrroloquinoléine quinone réduite,
dans laquelle l'analogue d'acide ascorbique est sélectionné dans le groupe consistant en l'acide ascorbique, l'acide rhamno-ascorbique, l'acide arabo-ascorbique, l'acide gluco-ascorbique, l'acide fuco-ascorbique, l'acide glucohepto-ascorbique, l'acide xylo-ascorbique, l'acide galacto-ascorbique, l'acide gulo-ascorbique, l'acide allo-ascorbique, l'acide érythro-ascorbique, l'acide 6-désoxyascorbique, et un ester et un sel de celui-ci.

2. Méthode selon la revendication 1, dans laquelle la solution de pyrroloquinoléine quinone ou d'un sel de celle-ci possède un pH de 2 à 3,5.

3. Méthode selon la revendication 1, dans laquelle le rapport molaire de l'analogue d'acide ascorbique par rapport à la pyrroloquinoléine quinone ou son sel est de 1/0,5 à 1000.

4. Méthode selon la revendication 1, comprenant en outre une étape de séparation de la pyrroloquinoléine quinone réduite depuis la solution.
